# EUROPEAN PATENT SPECIFICATION

(11) **EP 2 854 836 B1**
(45) Date of publication and mention of the grant of the patent: **04.04.2018**
(21) Application number: 13729224.9
(22) Date of filing: 05.06.2013
(51) Int. Cl.: A61K 38/18, A61P 25/00, A61P 25/22, A61P 25/24, A61P 25/28

(54) **NCAM PEPTIDE MIMETIC FOR USE IN TREATING MAJOR DEPRESSION DISORDER**
NCAM-PEPTIDMIMETIKA ZUR BEHANDLUNG VON DEPRESSION
MIMÉTIQUES PEPTIDIQUES DE N-CAM POUR LE TRAITEMENT DE LA DÉPRESSION MAJEURE

(30) Priority: 05.06.2012 US 201261655970 P; 14.03.2013 US 201361785374 P
(43) Date of publication of application: 08.04.2015
(73) Proprietor: The Board of Trustees of the Leland Stanford Junior University, Palo Alto, CA 94306-1106 (US)
(72) Inventor: AKIL, Huda, Ann Arbor, Michigan 48105 (US); WATSON, Stanley, Ann Arbor, Michigan 48105 (US); TURNER, Cortney, Willis, Michigan 48191 (US)
(74) Representative: J A Kemp
(86) International application number: PCT/US2013/044342
(87) International publication number: WO 2013/184824

(56) References cited:
- WO-A1-2009/059316
- WO-A2-2007/059064
- WO-A2-2007/110079
- WO-A2-2009/068042
- RETO BISAZ ET AL: "Causal evidence for the involvement of the neural cell adhesion molecule, NCAM, in chronic stress-induced cognitive impairments", HIPPOCAMPUS, vol. 21, no. 1, 23 January 2011 (2011-01-23), pages 56-71, XP055077889, ISSN: 1050-9631, DOI: 10.1002/hipo.20723

## Description

This application claims priority to U.S. provisional application no. 61/655,970, filed June 5, 2012, and U.S. provisional application no. 61/785,374, filed March 14, 2013.

### BACKGROUND OF THE INVENTION

Psychiatric disorders include any mental disorder or illness that interferes with the way a person behaves, interacts with others, and/or functions in daily life. *The Diagnostic and Statistical Manual (DSM) of Mental Disorders,* published by the American Psychiatric Association, classifies psychiatric disorders. The latest version, the DSM-5 (Fifth Edition), lists the following categories of mental disorders: adjustment disorders; anxiety disorders; delirium, dementia, amnestic, and other cognitive disorders; disorders usually first diagnosed in infancy, childhood or adolescence, such as learning disorders or communication disorders; dissociative disorders; eating disorders; factitious disorders; impulse-control disorders; mental disorders due to a general medical condition; mood disorders; other conditions of clinical importance; personality disorders; schizophrenia and other psychotic disorders; sexual and gender identity disorders; sleep disorders; somatoform disorders; and substance-related disorders. *See also,* http://www.dsm5.org.

The exact cause of most psychiatric disorders is not known. Mental health experts believe that psychiatric disorders typically result from a combination of genetic or inherited dispositions and a triggering event. Triggering events may include environmental factors, stresses of various kinds, and even physical health problems. Psychiatric disorders are very common in the United States. In fact, one-fifth of the American population suffers from some sort of mental disorder during any given year, according to the American Psychiatric Association.

Medication is widely used to treat a variety of psychiatric disorders. For example, antidepressants are used for the treatment of clinical depression as well as for anxiety and other disorders. Anxiolytics are used for anxiety disorders and related problems such as insomnia. Mood stabilizers are used primarily in bipolar disorder, mainly targeting mania rather than depression. Antipsychotics are used for psychotic disorders such as schizophrenia. Stimulants are commonly used, notably for attention deficit hyperactivity disorder (ADHD). However, despite the existence of an assortment of different medications, there is a need in the art for improved drugs (*e.g*., improved onset of action, increased efficacy, fewer adverse events, better adherence, *etc*.) to treat psychiatric disorders, particularly mood disorders (*e.g.,* depression), anxiety and symptoms thereof. The present invention satisfies this need and provides related advantages as well.

### BRIEF SUMMARY

In certain aspects, the present disclosure provides methods for treating or alleviating one or more symptoms of depression and/or anxiety in a subject comprising administering a therapeutically effective amount of an NCAM peptide mimetic to the subject. In some other aspects, the present disclosure provides methods for treating a neurological condition such as a psychiatric disorder (*e.g.,* mood disorders including depression and bipolar disorder, or anxiety) in a subject comprising administering a therapeutically effective amount of an NCAM peptide mimetic to the subject. The invention is defined in the claims.

Disclosed herein are NCAM peptide mimetics comprising compounds of Formula I or a pharmaceutically acceptable salt thereof:

(Zₙ₋Lₘ)_{q} (I),

wherein Z is an individually selected peptide comprising the amino acid sequence QQGKSKA, DVRRGIKKTD, or variants thereof; L is individually selected from the group consisting of optionally substituted lipophilic substituents, optionally substituted linkers, and optionally substituted spacers; n is an individually selected integer from about 1 to 6; m is an individually selected integer from about 0 to 6; and q is an individually selected integer from about 1 to 4.

The NCAM peptide mimetic for use in the invention has the following structure:

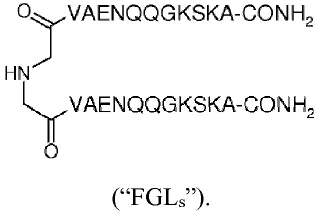

In one aspect of the disclosure, the NCAM peptide mimetic is a monomer and Z consists of the amino acid sequence EVYVVAENQQGKSKA ("FGLₘ").

In another aspect of the disclosure, the NCAM peptide mimetic has the following structure:

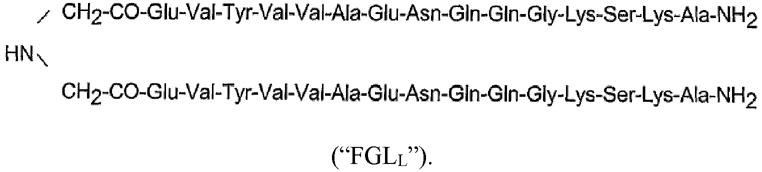

In another aspect of the disclosure, the NCAM peptide mimetic is a dendrimer having four copies of the amino acid sequence DVRRGIKKTD coupled to a three-lysine-containing backbone ("plannexin").

In some embodiments, an NCAM peptide mimetic is administered with a pharmaceutically acceptable carrier. Any of the pharmaceutical formulations of the invention may be administered in a single dose or in divided doses (*e.g.,* multiple sub-doses per day). In some other embodiments, an NCAM peptide mimetic is administered via a route selected from the group consisting of orally, nasally, by inhalation, topically, subcutaneously, intravenously, intraperitoneally, intrathecally, and intracerebroventricularly.

Other objects, features, and advantages of the present invention will be apparent to one of skill in the art from the following detailed description and figures.

### BRIEF DESCRIPTION OF THE DRAWINGS

Figure 1 shows the effect of FGLₘ administration on depression-like behavior as measured by the forced swim test (FST).
Figure 2 shows the effect of FGL_{L} administration on anxiety-like behavior as measured by the elevated plus maze (EPM).
Figure 3 shows the effect of FGL_{L} administration on depression-like behavior as measured by the FST.
Figure 4 shows the effect of FGL_{S} administration on depression-like behavior as measured by the FST.
Figure 5 shows another example of the effect of FGL_{S} administration on depression-like behavior as measured by the FST.
Figure 6 shows the effect of acute administration of plannexin on anxiety-like behavior as measured by the EPM.
Figure 7 shows the effect of chronic administration of plannexin on anxiety-like behavior as measured by the EPM.

### DETAILED DESCRIPTION

### I. Introduction

The present invention is based, in part, upon the surprising discovery that NCAM peptide mimetics are useful for treating or alleviating one or more symptoms of depression and/or anxiety in a subject in need thereof such as a subject having a neurological condition. In other aspects, the NCAM peptide mimetics described herein also find utility in treating a neurological condition such as a psychiatric disorder (*e.g.,* a mood disorder, *e.g.,* depression or bipolar disorder, or other psychiatric disorders such as, *e.g*., anxiety) in a subject in need thereof. In particular aspects, NCAM peptide mimetics such as, *e.g.,* FGLₘ, FGL_{L}, FGLₛ, and plannexin advantageously have the ability to modulate depression (*e.g.,* to produce an antidepressant effect) and/or anxiety (*e.g.,* to produce an anxiolytic effect) and therefore are useful for preventing, treating, and/or alleviating neurological conditions and/or one or more symptoms thereof such as depression and/or anxiety.

### II. Definitions

A "psychiatric disorder" or "mental disorder" or "mental illness" includes mood disorders (*e.g.,* depression of all forms and/or types, bipolar disorder, *etc.*), anxiety, anxiety disorders, psychotic disorders (*e.g.,* schizophrenia, personality disorders), as well as other mental disorders such as substance-related disorders, childhood disorders, dementia, autistic disorder, adjustment disorder, delirium, multi-infarct dementia, and Tourette's disorder as described in, *e.g.,* the Diagnostic and Statistical Manual (DSM) of Mental Disorders, Fifth Edition (DSM-5). Typically, such disorders have a complex genetic and/or a biochemical component.

A "mood disorder" as used herein includes disruption of feeling, tone or emotional state experienced by an individual for an extensive period of time. Mood disorders include, but are not limited to, depression (*i.e.,* depressive disorders), bipolar disorders, substance-induced mood disorders, alcohol-induced mood disorders, benzodiazepine-induced mood disorders, mood disorders due to general medical conditions, as well as many others. *See, e.g.,* DSM-5. There are many general medical conditions that can trigger mood episodes, including, but not limited to, neurological disorders (*e.g.,* dementias), metabolic disorders (*e.g.,* electrolyte disturbances), gastrointestinal diseases (*e.g.,* cirrhosis), endocrine disease (*e.g.,* thyroid abnormalities), cardiovascular disease (*e.g.,* heart attack), pulmonary disease (*e.g.,* chronic obstructive pulmonary disease), cancer, autoimmune diseases (*e.g.,* rheumatoid arthritis), and the like.

The term "depression" or "depressive disorder" includes a mood disorder involving any of the following symptoms: persistent sad, anxious, and/or "empty" mood; feelings of hopelessness and/or pessimism; feelings of guilt, worthlessness, and/or helplessness; loss of interest or pleasure in hobbies and activities that were once enjoyed, including sex; decreased energy, fatigue, and/or being "slowed down"; difficulty concentrating, remembering, and/or making decisions; insomnia, early-morning awakening, and/or oversleeping; loss of appetite and/or weight loss, overeating and/or weight gain; thoughts of death and/or suicide; suicide attempts; restlessness and/ or irritability; persistent physical symptoms that do not respond to treatment, such as headaches, digestive disorders, and/or chronic pain; and combinations thereof. *See, e.g.,* DSM-5. Non-limiting examples of depressive disorders include major depression disorder (MDD), atypical depression, melancholic depression, psychotic major depression or psychotic depression, catatonic depression, postpartum depression, seasonal affective disorder (SAD), chronic depression (dysthymia), double depression, depressive disorder not otherwise specified, depressive personality disorder (DPD), recurrent brief depression (RBD), minor depressive disorder (minor depression), premenstrual syndrome, premenstrual dysphoric disorder, depression caused by chronic medical conditions (*e.g.,* cancer, chronic pain, chemotherapy, chronic stress), and combinations thereof. Various subtypes of depression are described in, *e.g.,* DSM-5. The NCAM peptide mimetic is used in the invention in methods of treating or alleviating symptoms of major depression disorder (MDD).

"Bipolar disorder" includes a mood disorder characterized by alternating periods of extreme moods. A person with bipolar disorder experiences cycling of moods that usually swing from being overly elated or irritable (mania) to sad and hopeless (depression) and then back again, with periods of normal mood in between. Diagnosis of bipolar disorder is described in, *e.g.,* DSM-5. Bipolar disorders include bipolar disorder I (mania with or without major depression), bipolar disorder II (hypomania with major depression), and cyclothymia. *See, e.g.,* DSM-5. Bipolar disorder is also known as manic depression.

"Anxiety" includes a condition characterized by feelings of worry, nervousness, unease, and/or tension, typically about an imminent event or something with an uncertain outcome. Symptoms of anxiety include, without limitation, fear, panic, heart palpitations, shortness of breath, fatigue, nausea, headaches (*e.g.,* tension headaches), tachycardia, muscle weakness and/or tension, chest pain, stomach aches, pallor, sweating, trembling, pupillary dilation, panic attacks, and combinations thereof. *See, e.g.,* DSM-5. In certain instances, the methods of the present disclosure treat or alleviate one or more symptoms of anxiety. In other instances, the methods of the present disclosure treat anxiety or an anxiety disorder.

"A psychotic disorder" includes a condition that affects the mind, resulting in at least some loss of contact with reality. Symptoms of a psychotic disorder include, *e.g*., hallucinations, changed behavior that is not based on reality, delusions, and the like. *See, e.g.,* DSM-5. Schizophrenia, schizoaffective disorder, schizophreniform disorder, delusional disorder, brief psychotic disorder, substance-induced psychotic disorder, and shared psychotic disorder are non-limiting examples of psychotic disorders.

"Schizophrenia" includes a psychotic disorder involving a withdrawal from reality by an individual. Symptoms comprise for at least a part of a month two or more of the following symptoms: delusions (only one symptom is required if a delusion is bizarre, such as being abducted in a space ship from the sun); hallucinations (only one symptom is required if hallucinations are of at least two voices talking to one another or of a voice that keeps up a running commentary on the patient's thoughts or actions); disorganized speech (*e.g.,* frequent derailment or incoherence); grossly disorganized or catatonic behavior; or negative symptoms, *i.e.,* affective flattening, alogia, or avolition. Schizophrenia encompasses disorders such as, *e.g*., schizoaffective disorders. Diagnosis of schizophrenia is described in, *e.g.,* DSM-5. Types of schizophrenia include, *e.g.,* paranoid, disorganized, catatonic, undifferentiated, and residual. *See, e.g.,* DSM-5.

A "peptide mimetic" or "mimetic peptide" or "peptidomimetic" includes a peptide or a fragment or variant thereof that biologically mimics active determinants on proteins such as, but not limited to, receptors, hormones, cytokines, enzyme substrates, viruses and other biomolecules, and may antagonize, stimulate, and/or otherwise modulate the physiological activity of the natural ligands. *See, e.g.,* Fauchere, Adv. Drug Res. 15:29 (1986); Veber and Freidinger, TINS p. 392 (1985); and Evans et al., J. Med. Chem. 30:1229 (1987). In certain instances, peptide mimetics that are structurally similar to therapeutically useful peptides may be used to produce an equivalent or enhanced therapeutic or prophylactic effect. Peptide mimetics can be composed of natural amino acids, non-natural analogs of amino acids, or combinations thereof. Peptide mimetics can also incorporate any amount of natural amino acid conservative substitutions as long as such substitutions also do not substantially alter its structure and/or activity.

As used herein, an "NCAM peptide mimetic" or "NCAM mimetic peptide" or "NCAM peptidomimetic" includes a peptide or a fragment or variant thereof that mimics neural cell adhesion molecule (NCAM) homophilic binding (*e.g.,* NCAM binding to itself) and/or heterophilic binding (*e.g.,* NCAM binding to FGFR, other adhesion molecules, and various extracellular matrix components). In some instances, the NCAM peptide mimetic is an antagonist that interferes with, inhibits, or disrupts homophilic binding and/or heterophilic binding. In certain other instances, the NCAM peptide mimetic is an agonist that binds and activates or stimulates NCAM or an NCAM binding partner (*e.g.,* counter-receptor) such as FGFR. *See, e.g.,* Berezin et al., J. Mol. Neuroscience., 22:33-39 (2004).

The terms "FGL peptide" and "FG loop peptide" include a peptide or a fragment or variant thereof that contains a portion of the amino acid sequence in the second fibronectin type III (F3) module of NCAM (*see,* GenPept Accession No. P13591 for exemplary human NCAM amino acid sequence). In particular aspects, the amino acid sequence of the FGL peptide corresponds to a portion of the binding site of NCAM to a fibroblast growth factor receptor such as FGFR-1. Non-limiting examples of FGL peptides include FGLₘ, FGL_{L}, FGLₛ, and combinations thereof.

An "agonist" includes an agent that binds to a polypeptide and stimulates, increases, activates, facilitates, enhances activation, sensitizes or up regulates the activity or expression of the polypeptide.

An "antagonist" includes an agent that binds to a polypeptide and inhibits, partially or totally blocks stimulation, decreases, prevents, delays activation, inactivates, desensitizes, or down regulates the activity of the polypeptide.

"Inhibitors," "activators," and "modulators" of binding or activity as used herein include inhibitory, activating, or modulating molecules, respectively, identified using *in vitro* and *in vivo* assays for binding or activity, *e.g.,* ligands, agonists, antagonists, homo logs, and mimetics thereof. The term "modulator" includes inhibitors and activators. Inhibitors are agents that, *e.g.,* bind to a polypeptide and inhibit, partially or totally block stimulation or enzymatic activity, decrease, prevent, delay activation, inactivate, desensitize, or down regulate the activity of the polypeptide, *e.g.,* antagonists. Activators are agents that, *e.g.,* bind to, stimulate, increase, open, activate, facilitate, enhance activation or enzymatic activity, sensitize or up regulate the activity of a polypeptide, *e.g*., agonists. Modulators include naturally-occurring and synthetic ligands, antagonists, agonists, small chemical molecules and the like.

The term "isolated," when applied to a nucleic acid or protein, denotes that the nucleic acid or protein is essentially free of other cellular components with which it is associated in the natural state. It is preferably in a homogeneous state although it can be in either a dry or aqueous solution. Purity and homogeneity are typically determined using analytical chemistry techniques such as polyacrylamide gel electrophoresis or high performance liquid chromatography. A protein that is the predominant species present in a preparation is substantially purified. In particular, an isolated gene is separated from open reading frames that flank the gene and encode a protein other than the gene of interest. The term "purified" denotes that a nucleic acid or protein gives rise to essentially one band in an electrophoretic gel. Particularly, it means that the nucleic acid or protein is at least 85% pure, at least 95% pure, or at least 99% pure.

The term "nucleic acid" or "polynucleotide" includes deoxyribonucleotides or ribonucleotides and polymers thereof in either single- or double-stranded form. Unless specifically limited, the term encompasses nucleic acids containing known analogues of natural nucleotides that have similar binding properties as the reference nucleic acid and are metabolized in a manner similar to naturally occurring nucleotides. Unless otherwise indicated, a particular nucleic acid sequence also implicitly encompasses conservatively modified variants thereof (*e.g.,* degenerate codon substitutions), alleles, orthologs, SNPs, and complementary sequences as well as the sequence explicitly indicated. Specifically, degenerate codon substitutions may be achieved by generating sequences in which the third position of one or more selected (or all) codons is substituted with mixed-base and/or deoxyinosine residues (Batzer et al., Nucleic Acid Res. 19:5081 (1991); Ohtsuka et al., J. Biol. Chem. 260:2605-2608 (1985); and Cassol *et al.* (1992); Rossolini et al., Mol. Cell. Probes 8:91-98 (1994)). The term nucleic acid is used interchangeably with gene, cDNA, and mRNA encoded by a gene.

The terms "polypeptide," "peptide," and "protein" are used interchangeably herein to include a polymer of amino acid residues. The terms apply to amino acid polymers in which one or more amino acid residue is an artificial chemical mimetic of a corresponding naturally occurring amino acid, as well as to naturally occurring amino acid polymers and non-naturally occurring amino acid polymers. As used herein, the terms encompass amino acid chains of any length, including full-length proteins (*i*.*e*., antigens), wherein the amino acid residues are linked by covalent peptide bonds.

The term "amino acid" includes naturally occurring and synthetic amino acids, as well as amino acid analogs and amino acid mimetics that function in a manner similar to the naturally occurring amino acids. Naturally occurring amino acids are those encoded by the genetic code, as well as those amino acids that are later modified, *e.g*., hydroxyproline, γ-carboxyglutamate, and O-phosphoserine. Amino acid analogs include compounds that have the same basic chemical structure as a naturally occurring amino acid, *i*.*e*., an α carbon that is bound to a hydrogen, a carboxyl group, an amino group, and an R group, *e.g*., homoserine, norleucine, methionine sulfoxide, methionine methyl sulfonium. Such analogs have modified R groups (*e.g.,* norleucine) or modified peptide backbones, but retain the same basic chemical structure as a naturally occurring amino acid. "Amino acid mimetics" include chemical compounds that have a structure that is different from the general chemical structure of an amino acid, but that functions in a manner similar to a naturally occurring amino acid.

Amino acids may be referred to herein by either the commonly known three letter symbols or by the one-letter symbols recommended by the IUPAC-IUB Biochemical Nomenclature Commission. Nucleotides, likewise, may be referred to by their commonly accepted single-letter codes.

"Conservatively modified variants" applies to both amino acid and nucleic acid sequences. With respect to particular nucleic acid sequences, "conservatively modified variants" include those nucleic acids that encode identical or essentially identical amino acid sequences, or where the nucleic acid does not encode an amino acid sequence, to essentially identical sequences. Because of the degeneracy of the genetic code, a large number of functionally identical nucleic acids encode any given protein. For instance, the codons GCA, GCC, GCG and GCU all encode the amino acid alanine. Thus, at every position where an alanine is specified by a codon, the codon can be altered to any of the corresponding codons described without altering the encoded polypeptide. Such nucleic acid variations are "silent variations," which are one species of conservatively modified variations. Every nucleic acid sequence herein that encodes a polypeptide also describes every possible silent variation of the nucleic acid. One of skill will recognize that each codon in a nucleic acid (except AUG, which is ordinarily the only codon for methionine, and TGG, which is ordinarily the only codon for tryptophan) can be modified to yield a functionally identical molecule. Accordingly, each silent variation of a nucleic acid that encodes a polypeptide is implicit in each described sequence.

As to amino acid sequences, one of skill will recognize that individual substitutions, deletions and/or additions to a nucleic acid, peptide, polypeptide, or protein sequence which alters, adds or deletes a single amino acid or a small percentage of amino acids in the encoded sequence is a "conservatively modified variant" where the alteration results in the substitution of an amino acid with a chemically similar amino acid. Conservative substitution tables providing functionally similar amino acids are well known in the art. Such conservatively modified variants are in addition to and do not exclude polymorphic variants, interspecies homologs, and/or alleles.

The following eight groups each contain amino acids that are conservative substitutions for one another:
1) Alanine (A), Glycine (G);
2) Aspartic acid (D), Glutamic acid (E);
3) Asparagine (N), Glutamine (Q);
4) Arginine (R), Lysine (K);
5) Isoleucine (I), Leucine (L), Methionine (M), Valine (V);
6) Phenylalanine (F), Tyrosine (Y), Tryptophan (W);
7) Serine (S), Threonine (T); and
8) Cysteine (C), Methionine (M)
(*see, e.g.,* Creighton, Proteins (1984)).

The term "recombinant" when used with reference, *e.g.,* to a cell, or nucleic acid, protein, or vector, indicates that the cell, nucleic acid, protein or vector, has been modified by the introduction of a heterologous nucleic acid or protein or the alteration of a native nucleic acid or protein, or that the cell is derived from a cell so modified. For example, recombinant cells express genes that are not found within the native (non-recombinant) form of the cell or express native genes that are otherwise abnormally expressed, under-expressed, or not expressed at all.

The term "heterologous" when used with reference to portions of a nucleic acid indicates that the nucleic acid comprises two or more subsequences that are not found in the same relationship to each other in nature. For instance, the nucleic acid is typically recombinantly produced, having two or more sequences from unrelated genes arranged to make a new functional nucleic acid, *e.g.,* a promoter from one source and a coding region from another source. Similarly, a heterologous protein indicates that the protein comprises two or more subsequences that are not found in the same relationship to each other in nature (*e.g.,* a fusion protein).

An "expression vector" includes a nucleic acid construct, generated recombinantly or synthetically, with a series of specified nucleic acid elements that permit transcription of a particular nucleic acid in a host cell. The expression vector can be part of a plasmid, virus, or nucleic acid fragment. Typically, the expression vector includes a nucleic acid to be transcribed operably linked to a promoter.

One who is "predisposed for a psychiatric or mental disorder" as used herein means a person who has an inclination or a higher likelihood of developing a psychiatric or mental disorder when compared to an average person in the general population.

A "therapeutically effective amount" includes an amount or quantity effective, at dosages and for periods of time necessary, to achieve the desired therapeutic or prophylactic result.

### III. Detailed Description

In certain aspects, the present disclosure provides methods for treating or alleviating one or more symptoms of depression and/or anxiety in a subject (in need thereof) comprising administering a therapeutically effective amount of an NCAM peptide mimetic to the subject.

Non-limiting examples of symptoms of depression that can be treated in accordance with the present invention include persistent sad, anxious, and/or "empty" mood; feelings of hopelessness and/or pessimism; feelings of guilt, worthlessness, and/or helplessness; loss of interest or pleasure in hobbies and activities that were once enjoyed, including sex; decreased energy, fatigue, and/or being "slowed down"; difficulty concentrating, remembering, and/or making decisions; insomnia, early-morning awakening, and/or oversleeping; loss of appetite and/or weight loss, overeating and/or weight gain; thoughts of death and/or suicide; suicide attempts; restlessness and/ or irritability; persistent physical symptoms that do not respond to treatment, such as headaches, digestive disorders, and/or chronic pain; and combinations thereof. In particular embodiments, the presence, severity, frequency, and/or duration of these symptoms vary on a case by case basis. In some embodiments, a subject may have at least one, at least two, at least three, at least four, or at least five of these symptoms.

Non-limiting examples of symptoms of anxiety that can be treated in accordance with the present disclosure include fear, panic, heart palpitations, shortness of breath, fatigue, nausea, headaches (*e.g.,* tension headaches), tachycardia, muscle weakness and/or tension, chest pain, stomach aches, pallor, sweating, trembling, pupillary dilation, panic attacks, and combinations thereof. In particular aspects of the disclosure, the presence, severity, frequency, and/or duration of these symptoms vary on a case by case basis. In some aspects, a subject may have at least one, at least two, at least three, at least four, or at least five of these symptoms.

In certain aspects, the symptoms of depression and/or anxiety are associated with a neurological condition such as a psychiatric disorder (*e.g.,* a mood disorder such as depression, or anxiety). In some aspects, a subject may have at least one, at least two, at least three, at least four, at least five, at least six, at least seven, at least eight, at least nine, at least ten, at least fifteen, at least twenty, or more symptoms of depression, anxiety, or combinations thereof.

In particular aspects, the symptoms of depression and/or anxiety are observed in or associated with a neurological condition such as, *e.g.,* a psychiatric disorder, a learning disorder, autistic disorder, attention-deficit hyperactivity disorder, Tourette's syndrome, phobia, post-traumatic stress disorder, dementia, AIDS dementia, Alzheimer's disease, Parkinson's disease, Huntington's disease, spasticity, myoclonus, muscle spasm, substance abuse disorder, urinary incontinence, pain such as chronic pain, and combinations thereof.

In certain aspects, the symptoms of depression and/or anxiety are observed in or associated with a psychiatric disorder including, but not limited to, a mood disorder (*e.g.,* depression, bipolar disorder), anxiety, an anxiety disorder, a psychotic disorder, other related conditions, and combinations thereof.

Non-limiting examples of mood disorders in which one or more symptoms of depression and/or anxiety can be treated in accordance with the methods described herein include depression (*i.e*., depressive disorders), bipolar disorders, substance-induced mood disorders, alcohol-induced mood disorders, benzodiazepine-induced mood disorders, mood disorders due to general medical conditions, and combinations thereof. Examples of various forms of depression include, without limitation, major depression disorder (MDD), atypical depression, melancholic depression, psychotic depression, catatonic depression, postpartum depression, seasonal affective disorder (SAD), chronic depression (dysthymia), double depression, depressive disorder not otherwise specified, depressive personality disorder (DPD), recurrent brief depression (RBD), minor depressive disorder (minor depression), premenstrual syndrome, premenstrual dysphoric disorder, depression caused by chronic medical conditions (*e.g.,* cancer, chronic pain, chemotherapy, chronic stress, and the like), and combinations thereof.

Non-limiting examples of anxiety disorders in which one or more symptoms of depression and/or anxiety can be treated in accordance with the methods described herein include generalized anxiety disorder, obsessive-compulsive disorder, panic disorder, agoraphobia, posttraumatic stress disorder (PTSD), social anxiety disorder, and combinations thereof.

Non-limiting examples of psychotic disorders in which one or more symptoms of depression and/or anxiety can be treated in accordance with the methods described herein include schizophrenia and all forms thereof (*e.g.,* catatonic, subchronic, chronic, with acute exacerbation, in remission, unspecified, disorganized, paranoid, residual, and/or undifferentiated), schizoaffective disorder, schizophreniform disorder, personality disorders (*e.g.,* paranoid, schizoid, schizotypal, antisocial, borderline, *etc.*), psychosis (*e.g.,* paranoid psychosis, catatonic psychosis, delusional psychosis, *etc.*), a substance-induced psychotic disorder, and combinations thereof.

In certain instances, the subject has been diagnosed with one or more symptoms of depression and/or anxiety. In preferred embodiments, the subject is a human.

Methods for treating a neurological condition such as a psychiatric disorder (*e.g.,* a mood disorder such as depression, or anxiety) in a subject (in need thereof) comprising administering a therapeutically effective amount of an NCAM peptide mimetic to the subject are also described.

Non-limiting examples of neurological conditions that can be treated in accordance with the disclosed methods include a psychiatric disorder, a learning disorder, autistic disorder, attention-deficit hyperactivity disorder, Tourette's syndrome, phobia, post-traumatic stress disorder, dementia, AIDS dementia, Alzheimer's disease, Parkinson's disease, Huntington's disease, spasticity, myoclonus, muscle spasm, substance abuse disorder, urinary incontinence, pain such as chronic pain, and combinations thereof.

In particular aspects of the disclosure, the psychiatric disorder comprises a mood disorder (e.g., depression, bipolar disorder), anxiety, an anxiety disorder, a psychotic disorder, other related conditions, and combinations thereof.

Non-limiting examples of mood disorders that can be treated in accordance with the present disclosure include depression (*i.e.,* depressive disorders), bipolar disorders, substance-induced mood disorders, alcohol-induced mood disorders, benzodiazepine-induced mood disorders, mood disorders due to general medical conditions, and combinations thereof. Examples of various forms of depression include, without limitation, major depression disorder (MDD), atypical depression, melancholic depression, psychotic depression, catatonic depression, postpartum depression, seasonal affective disorder (SAD), chronic depression (dysthymia), double depression, depressive disorder not otherwise specified, depressive personality disorder (DPD), recurrent brief depression (RBD), minor depressive disorder (minor depression), premenstrual syndrome, premenstrual dysphoric disorder, depression caused by chronic medical conditions (*e.g.,* cancer, chronic pain, chemotherapy, chronic stress, and the like), and combinations thereof.

Non-limiting examples of anxiety disorders that can be treated in accordance with the present disclosure include generalized anxiety disorder, obsessive-compulsive disorder, panic disorder, agoraphobia, posttraumatic stress disorder (PTSD), social anxiety disorder, and combinations thereof.

Non-limiting examples of psychotic disorders that can be treated in accordance with the present disclosure include schizophrenia and all forms thereof (*e.g.,* catatonic, subchronic, chronic, with acute exacerbation, in remission, unspecified, disorganized, paranoid, residual, and/or undifferentiated), schizoaffective disorder, schizophreniform disorder, personality disorders (*e.g.,* paranoid, schizoid, schizotypal, antisocial, borderline, *etc.*), psychosis (*e.g.,* paranoid psychosis, catatonic psychosis, delusional psychosis, *etc.*), a substance-induced psychotic disorder, and combinations thereof.

In certain instances, the subject has been diagnosed with a neurological condition such as a psychiatric disorder (*e.g.,* a mood disorder such as depression, or anxiety). In preferred embodiments, the subject is a human.

Described herein are NCAM peptide mimetics comprising a compound of Formula I or a pharmaceutically acceptable salt thereof:

(Zₙ-Lₘ)_{q} (I),

wherein Z is an individually selected peptide comprising the amino acid sequence QQGKSKA, DVRRGIKKTD, or variants thereof; L is individually selected from the group consisting of optionally substituted lipophilic substituents, optionally substituted linkers, and optionally substituted spacers; n is an individually selected integer from about 1 to 6; m is an individually selected integer from about 0 to 6; and q is an individually selected integer from about 1 to 4.

In some instances, Z independently comprises at least about 7, 8, 9, 10, 11, 12, 13, 14, 15, 16, 17, 18, 19, 20, 21, 22, 23, 24, or 25 amino acid residues. In certain instances, the compound is a monomer. In certain other instances, the compound is a multimer. Examples of multimeric compounds include, but are not limited to, a dimer, a tetramer, and a dendrimer. In the multimeric compounds, Z can be the same peptide or Z can comprise different peptides.

In certain aspects of the disclosure, Z is independently selected from the group consisting of VAENQQGKSKA, EVYVVAENQQGKSKA, and variants thereof.

In some aspects of the disclosure, the compound is a monomer or a dimer and Z independently comprises or consists of the amino acid sequence QQGKSKA and optionally 1, 2, 3, 4, 5, 6, or 7 flanking and contiguous amino acids of the amino acid sequence VYVVAEN at the N-terminus thereof (*e.g.,* NQQGKSKA, ENQQGKSKA, AENQQGKSKA, VAENQQGKSKA, VVAENQQGKSKA, YVVAENQQGKSKA, VYVVAENQQGKSKA), and optionally 1, 2, 3, 4, 5, 6, 7, 8, 9, 10, or more flanking and contiguous amino acids at the C-terminus thereof from an NCAM polypeptide, or variants thereof (e.g., amino acid sequences having at least about 50%, 55%, 60%, 65%, 70%, 75%, 80%, 85%, 90%, 95%, or greater identity thereto). In some instances, the compound is a dimer and each Z consists of the amino acid sequence VAENQQGKSKA.

The compound for use in the method of the invention has the following structure:

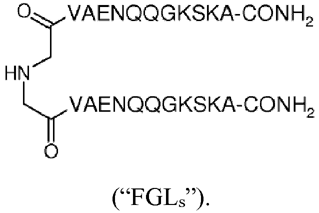

In certain aspects, the compound is a monomer and Z comprises or consists of the amino acid sequence EVYVVAENQQGKSKA. In other aspects, the compound is a dimer and each Z independently comprises or consists of EVYVVAENQQGKSKA.

In one aspect, the compound is a monomer and Z consists of the amino acid sequence EVYVVAENQQGKSKA ("FGLₘ"). In another aspect, the compound is a dimer and each Z consists of the amino acid sequence EVYVVAENQQGKSKA.

In one particular aspect, the compound has the following structure:

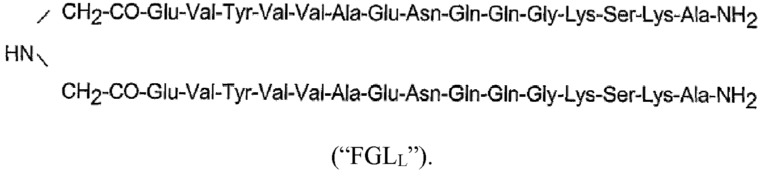

In another particular aspect, the compound is a dendrimer having four copies of the amino acid sequence DVRRGIKKTD coupled to a three-lysine-containing backbone ("plannexin").

In some embodiments, an NCAM peptide mimetic is administered with a pharmaceutically acceptable carrier. In certain embodiments, an NCAM peptide mimetic is administered via a route selected from orally, nasally, by inhalation, topically, subcutaneously, intravenously, intraperitoneally, intrathecally, and intracerebroventricularly.

In certain embodiments, a therapeutically effective amount of an NCAM peptide mimetic comprises a dose of about 0.5, 1, 2, 5, 10, 20, 40, 50, 75, 100, 125, 150, 175, 200, 250, 300, 400, 500, 600, 700, 750, 800, or 900 mg, or about 1, 1.5, 2, 2.5, 3, 3.5, 4, 4.5, 5, 5.5, 6, 6.5, 7, 7.5, 8, 8.5, 9, 9.5, or 10 grams (g) of the peptide, *e.g.,* per day. In certain other embodiments, a therapeutically effective amount of an NCAM peptide mimetic comprises a dose of between about 0.001 mg/kg to about 1,000 mg/kg, about 0.01 mg/kg to about 1,000 mg/kg, about 0.1 mg/kg to about 1,000 mg/kg, about 0.1 mg/kg to about 100 mg/kg, about 1 to about 10 mg/kg, about 1 mg/kg, about 5 mg/kg, about 10 mg/kg, about 50 mg/kg, about 100 mg/kg, about 200 mg/kg, about 300 mg/kg, about 400 mg/kg, about 500 mg/kg, about 600 mg/kg, about 700 mg/kg, about 800 mg/kg, about 900 mg/kg, or about 1,000 mg/kg of the peptide, *e.g.,* per day. The desired dose may be conveniently administered in a single dose, or as multiple doses administered at appropriate intervals, for example as two, three, four, or more sub-doses per day.

In particular embodiments, a therapeutically effective amount of an NCAM peptide mimetic is administered acutely, *e.g.,* as a single dose or as multiple doses over a short period of time (*e.g.,* over a span of less than about 24 hours), to a subject. In certain instances, the pharmaceutical compositions are acutely administered intravenously, intranasally, orally, by inhalation, or subcutaneously as a single dose or as multiple doses over a short period of time. Non-limiting examples of doses for providing a therapeutically effective amount of a peptide are described above.

In particular embodiments, a therapeutically effective amount of an NCAM peptide mimetic is administered chronically, *e.g.,* as repeated doses spanning hours (*e.g.,* every 24, 48, or 72 hours), days, weeks, months, or years, to a subject. As non-limiting examples, the pharmaceutical compositions comprising the peptides described herein can be administered at least 1, 2, 3, 4, 5, 6, 7, 8, 9, 10, or more times daily for at least 1, 2, 3, 4, 5, 6, or 7 days a week for a period of at least 1, 2, 3, 4, 5, 6, 7, 8, 9, 10, 11, 12, 13, 14, 15, 16, 17, 18, 19, 20, or more weeks or months. In certain instances, a rest period ranging from a few days (*e.g.,* 1, 2, 3, 4, 5, 6, 7, 8, 9, 10, or more days) to a few weeks (*e.g.,* 1, 2, 3, 4, 5, 6, 7, 8, 9, 10, or more weeks) can be introduced to improve the tolerability and/or efficacy of the treatment. In one exemplary embodiment, the pharmaceutical compositions are administered once daily, three times a week (*e.g.,* Monday, Wednesday, and Friday) for 2 weeks. In certain instances, the pharmaceutical compositions are chronically administered intravenously, intranasally, orally, by inhalation, or subcutaneously as repeated doses. Non-limiting examples of doses for providing a therapeutically effective amount of a peptide are described above.

In certain aspects, a therapeutically effective amount of an NCAM peptide mimetic comprises an amount that is sufficient to decrease anxiety-like behavior (anxiolytic effect) in a subject for treating a neurological condition such as a psychiatric disorder (*e.g.,* a mood disorder such as depression, or anxiety) and/or for treating one or more symptoms of depression and/or anxiety. In certain other apsects, a therapeutically effective amount of an NCAM peptide mimetic comprises an amount that is sufficient to decrease depression (antidepressant effect) in a subject for treating a neurological condition such as a psychiatric disorder (*e.g.,* a mood disorder such as depression, or anxiety) and/or for treating one or more symptoms of depression and/or anxiety. In certain aspects, the severity of the anxiety or depression is decreased (*e.g.,* a reduction in the number and/or severity of one or more symptoms) in the subject after administration of the NCAM peptide mimetic relative (or compared) to the severity of the anxiety or depression in the subject prior to administration of the NCAM peptide mimetic.

In certain aspects, a therapeutically effective amount of an NCAM peptide mimetic comprises an amount that is sufficient to increase anxiety-like behavior (anxiogenic effect) in a subject (*e.g.,* for treating a personality disorder such as an antisocial personality disorder or for reducing risk-taking behavior). In other aspects, a therapeutically effective amount of an NCAM peptide mimetic comprises an amount that is sufficient to increase depression-like behavior in the subject (*e.g.,* for treating a personality disorder such as an antisocial personality disorder or for reducing risk-taking behavior). In particular aspects, the severity of the anxiety or depression is increased (*e.g.,* an elevation in the number and/or severity of one or more symptoms) in the subject after administration of the NCAM peptide mimetic relative (or compared) to the severity of the anxiety or depression in the subject prior to administration of the NCAM peptide mimetic.

In some instances, a therapeutically effective amount of an NCAM peptide mimetic comprises an amount that is sufficient to relieve or substantially relieve a subject of at least one symptom of depression or anxiety for about 2 weeks or less, about 1 week or less, about 1 day or less, about 1 hour or less, about 30 minutes or less, or about 15 minutes or less after the administration. In other instances, a therapeutically effective amount of an NCAM peptide mimetic comprises an amount that is sufficient to relieve or substantially relieve a subject of at least one symptom of depression or anxiety for about 15 minutes or more, about 30 minutes or more, about 1 hour or more, about 1 day or more, about 1 week or more, or about 2 weeks or more after the administration.

In some instances, a therapeutically effective amount of an NCAM peptide mimetic comprises an amount that is sufficient to relieve or substantially relieve a subject of at least one symptom of depression or anxiety substantially earlier after the (first) administration of the NCAM peptide mimetic, *e.g*., as compared to the same subject administered a different antidepressant or anxiolytic compound. In certain embodiments, the subject is substantially relieved of one or more symptoms of depression or anxiety within about 1 day to about 21 days, about 1 day to about 14 days, about 1 day to about 7 days, about 12 hours to about 1 day, or about 1 hour to about 12 hours after administration of an NCAM peptide mimetic.

In certain embodiments, a therapeutically effective amount of an NCAM peptide mimetic comprises an amount that is sufficient to produce an antidepressant effect without essentially any dissociative side-effects. In other embodiments, a therapeutically effective amount of an NCAM peptide mimetic comprises an amount that is sufficient to produce an antidepressant effect with essentially no sedation. In other embodiments, a therapeutically effective amount of an NCAM peptide mimetic comprises an amount that does not have abuse potential (*e.g.,* may not be habit-forming).

In certain instances, an NCAM peptide mimetic used in the present invention provides improved blood-brain barrier (BBB) penetration and is capable of readily crossing the BBB. In other instances, an NCAM peptide mimetic used in the present invention provides improved *in vivo* potency and/or brain level concentration, *e.g.,* relative to plasma levels. In certain other instances, an NCAM peptide mimetic used in the present invention has a wide therapeutic index, provides a high therapeutic index, or combinations thereof.

### IV. Peptides

In some aspects, the peptides of the disclosure (*e.g.,* NCAM peptide mimetics) comprise a compound of Formula I or a pharmaceutically acceptable salt thereof:

(Zₙ-Lₘ)_{q} (I),

wherein Z is an individually selected peptide comprising the amino acid sequence QQGKSKA, DVRRGIKKTD, or variants thereof (*e.g.,* amino acid sequences having at least about 50%, 55%, 60%, 65%, 70%, 75%, 80%, 85%, 90%, or 95% identity); L is individually selected from the group consisting of optionally substituted lipophilic substituents, optionally substituted linkers, and optionally substituted spacers; n is an individually selected integer from about 1 to 6 (*e.g.,* 1, 2, 3, 4, 5, or 6); m is an individually selected integer from about 0 to 6 (*e.g.,* 0, 1, 2, 3, 4, 5, or 6); and q is an individually selected integer from about 1 to 4 (*e.g.,* 1, 2, 3, or 4).

Each peptide (Z) in the compound of Formula I may independently comprise about 7-160, 7-150, 7-140, 7-130, 7-120, 7-110, 7-100, 7-90, 7-80, 7-70, 7-60, 7-50, 7-40, 7-30, 7-20, 7-15, or 7-10 amino acid residues. In some aspects, each peptide (Z) independently comprises about 10-100, 10-90, 10-80, 10-70, 10-60, 10-50, 10-40, 10-30, 10-25, 10-20, or 10-15 amino acid residues. In other aspects, each peptide (Z) independently comprises at least about 7, 8, 9, 10, 11, 12, 13, 14, 15, 16, 17, 18, 19, 20, 21, 22, 23, 24, or 25 amino acid residues. In certain aspects, Z comprises the amino acid sequence QQGKSKA, DVRRGIKKTD, or variants thereof, as well as at least about 1, 2, 3, 4, 5, 6, 7, 8, 9, 10, 11, 12, 13, 14, 15, 16, 17, 18, 19, 20, 21, 22, 23, 24, 25, or more flanking and contiguous amino acids from an NCAM polypeptide (*see,* GenPept Acc. No. P13591 for an exemplary human NCAM amino acid sequence).

In particular aspects, each peptide (Z) independently comprises the amino acid sequence VAENQQGKSKA, EVYVVAENQQGKSKA, or variants thereof (*e.g.,* amino acid sequences having at least about 50%, 55%, 60%, 65%, 70%, 75%, 80%, 85%, 90%, or 95% identity). In certain aspects, each peptide (Z) independently comprises the amino acid sequence VAENQQGKSKA, EVYVVAENQQGKSKA, or variants thereof, as well as at least about 1, 2, 3, 4, 5, 6, 7, 8, 9, 10, 11, 12, 13, 14, 15, 16, 17, 18, 19, 20, 21, 22, 23, 24, 25, or more flanking and contiguous amino acids from an NCAM polypeptide (*see,* GenPept Acc. No. P13591 for an exemplary human NCAM amino acid sequence). In some instances, all of the peptides (Z) comprise the identical amino acid sequence.

Each peptide (Z) may be connected to another peptide sequence by a chemical bond in a fusion protein or the amino acid sequences may be connected to each other through a linker group. In some aspects, a peptide sequence may be formulated as an oligomer (multimer) of monomers, wherein each monomer comprises a peptide sequence as defined above. In particular aspects, multimeric peptides such as dendrimers may form conformational determinants or clusters due to the presence of multiple flexible peptide monomers. In one aspect, the compound is a dimer. In another aspect, the compound is a trimer or a tetramer. In one aspect, the compound is a dendrimer, such as four peptides linked to a lysine backbone, or coupled to a polymer carrier, for example a protein carrier, such as BSA. Polymerization such as repetitive sequences or attachment to various carriers are well known in the art, *e.g.,* lysine backbones, such as lysine dendrimers carrying 4 peptides, 8 peptides, 16 peptides, or 32 peptides. Other carriers may be lipophilic dendrimers, or micelle-like carriers formed by lipophilic derivatives, or starburst (star-like) carbon chain polymer conjugates.

In certain instances, a multimeric compound may be a polymer comprising two or more identical or different peptide sequences. For instance, the compound for use in the method of the invention comprises two identical amino acid sequences. Also disclosed are compounds comprising four identical copies of an amino acid sequence. In other aspects, the compound may comprise two or more different amino acid sequences.

Non-limiting examples of additional peptide sequences (Z) suitable for use in the present disclosure include those amino acid sequences described in PCT Patent Publication Nos. WO 2009/068042, WO 2008/022645, WO 2007/045243, WO 2005/014623, WO 2005/030804, WO 2005/123759, WO 2004/056865, WO 03/016351, WO 03/020749, and WO 02/47719.

In certain aspects, the peptide sequences in the multimeric compounds of the disclosure are connected to each other through a linker of Formula II:

X[(A)nCOOH][(B)mCOOH] (II),

wherein n and m independently are an integer of from about 1 to 20; X is selected from the group consisting of HN, H₂N(CR₂)pCR, RHN(CR₂)pCR, HO(CR₂)pCR, HS(CR₂)pCR, halogen-(CR₂)pCR, HOOC(CR₂)pCR, ROOC(CR₂)pCR, HCO(CR₂)pCR, RCO(CR₂)pCR, [HOOC(A)n][HOOC(B)m]CR(CR₂)pCR, H2N(CR₂)p, RHN(CR₂)p, HO(CR₂)p, HS(CR₂)p, halogen-(CR₂)p, HOOC(CR₂)p, ROOC(CR₂)p, HCO(CR₂)p, RCO(CR₂)p, and [HOOC(A)n][HOOC(B)m](CR₂)p; p is 0 or integer of from about 1 to 20; and A and B independently are a substituted or unsubstituted C₁₋₁₀ alkyl, a substituted or unsubstituted C₂₋₁₀ alkenyl, a substituted or unsubstituted cyclic moiety, a substituted or unsubstituted heterocyclic moiety, a substituted or unsubstituted aromatic moiety, or A and B together form a substituted or unsubstituted cyclic moiety, substituted or unsubstituted heterocyclic moiety, or substituted or unsubstituted aromatic moiety.

The peptide for use in the invention (*e.g.,* NCAM peptide mimetic) is a dimeric compound having the following structure:

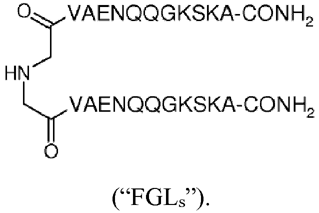

In another particular aspect, the peptide of the disclosure (*e.g.,* NCAM peptide mimetic) is a monomeric compound that comprises or consists of the amino acid sequence EVYVVAENQQGKSKA ("FGLₘ").

In another particular aspect, the peptide of the disclosure (*e.g.,* NCAM peptide mimetic) is a dimeric compound having the following structure:

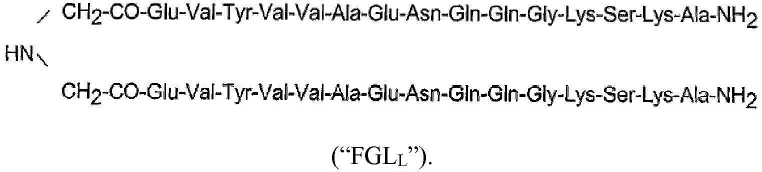

In another particular aspect, the peptide of the disclosure (*e.g.,* NCAM peptide mimetic) is a tetrameric dendrimer compound having four copies of the amino acid sequence DVRRGIKKTD coupled to a three-lysine-containing backbone ("plannexin"). *See, e.g.,* Kraev et al., 2011, PLoS ONE, 6(8):e23433; and Køhler et al., 2010, J Neurosci Res., Aug 1;88(10):2165-76.

### V. Preparation of Peptides for use in the Invention

In some embodiments, the peptides for use in the invention (*e.g.,* NCAM peptide mimetics) are produced by use of recombinant DNA technologies.

In certain instances, the present invention relies on routine techniques in the field of recombinant genetics. Basic texts disclosing the general methods of use in this invention include Sambrook et al., Molecular Cloning, A Laboratory Manual (3rd ed. 2001); Kriegler, Gene Transfer and Expression: A Laboratory Manual (1990); and Current Protocols in Molecular Biology (Ausubel et al., eds., 1994)).

The DNA sequence encoding a peptide or the corresponding full-length protein from which the peptide originates may be prepared synthetically by established standard methods, *e.g.,* the phosphoamidine method described by Beaucage and Caruthers, 1981, Tetrahedron Lett. 22:1859-1869, or the method described by Matthes et al., 1984, EMBO J. 3:801-805. According to the phosphoamidine method, oligonucleotides are synthesised, *e.g*., in an automatic DNA synthesiser, purified, annealed, ligated and cloned in suitable vectors.

The DNA sequence encoding a peptide may also be prepared by fragmentation of the DNA sequences encoding the corresponding full-length protein of peptide origin, using DNAase I according to a standard protocol (*e.g.,* Sambrook et al., Molecular Cloning, A Laboratory Manual (3rd ed. 2001)). The DNA encoding the full-length proteins may alternatively be fragmented using specific restriction endonucleases. The fragments of DNA can then be further purified using standard procedures described in, *e.g.,* Sambrook et al., Molecular Cloning, A Laboratory Manual (3rd ed. 2001).

The DNA sequence encoding a full-length protein may also be of genomic or cDNA origin, *e.g*., obtained by preparing a genomic or cDNA library and screening for DNA sequences coding for all or part of the full-length protein by hybridization using synthetic oligonucleotide probes in accordance with standard techniques (*e.g.,* Sambrook *et al*., *supra*). The DNA sequence may also be prepared by the polymerase chain reaction using specific primers, *e.g.,* as described in U.S. Patent No. 4,683,202 and Saiki et al., 1988, Science 239:487-491.

In certain embodiments, the DNA sequence is then inserted into a recombinant expression vector, which may be any vector, which may conveniently be subjected to recombinant DNA procedures. The choice of vector may depend on the host cell into which it is to be introduced. Thus, the vector may be an autonomously replicating vector, *e.g.,* a vector that exists as an extrachromosomal entity, the replication of which is independent of chromosomal replication, *e.g*., a plasmid. Alternatively, the vector may be one which, when introduced into a host cell, is integrated into the host cell genome and replicated together with the chromosome(s) into which it has been integrated.

In the vector, the DNA sequence encoding a peptide or a full-length protein should be operably connected to a suitable promoter sequence. The promoter may be any DNA sequence which shows transcriptional activity in the host cell of choice and may be derived from genes encoding proteins either homologous or heterologous to the host cell. Non-limiting examples of suitable promoters for directing the transcription of the coding DNA sequence in mammalian cells include the SV 40 promoter (Subramani et al., 1981, Mol. Cell Biol. 1:854-864), the MT-1 (metallothionein gene) promoter (Palmiter et al., 1983, Science 222: 809-814) and the adenovirus 2 major late promoter. A suitable promoter for use in insect cells is the polyhedrin promoter (Vasuvedan et al., 1992, FEBS Lett. 311:7-11). Suitable promoters for use in yeast host cells include promoters from yeast glycolytic genes (Hitzeman et al., 1980, J. Biol. Chem. 255:12073-12080; Alber and Kawasaki, 1982, J. Mol. Appl. Gen. 1: 419-434) and alcohol dehydrogenase genes (Young et al., 1982, in Genetic Engineering of Microorganisms for Chemicals, Hollaender et al, eds., Plenum Press, New York), and the TPI1 (U.S. Patent No. 4,599,311) or ADH2-4c (Russell et al., 1983, Nature 304:652-654) promoters. Suitable promoters for use in filamentous fungus host cells include, but are not limited to, the ADH3 promoter (McKnight et al., 1985, EMBO J. 4:2093-2099) and the tpiA promoter.

The coding DNA sequence may also be operably connected to a suitable terminator, such as the human growth hormone terminator (Palmiter et al., *supra*) or (for fungal hosts) the TPI1 (Alber and Kawasaki, *supra*) or ADH3 (McKnight et al., *supra*) promoters. The vector may further comprise elements such as polyadenylation signals (*e.g.,* from SV 40 or the adenovirus 5 Elb region), transcriptional enhancer sequences (*e.g.,* the SV 40 enhancer), and translational enhancer sequences (*e.g.,* the ones encoding adenovirus VA RNAs).

The recombinant expression vector may further comprise a DNA sequence enabling the vector to replicate in the host cell in question. An example of such a sequence (when the host cell is a mammalian cell) includes the SV 40 origin of replication. The vector may also comprise a selectable marker, *e.g.,* a gene the product of which complements a defect in the host cell, such as the gene coding for dihydrofolate reductase (DHFR) or one which confers resistance to a drug, *e.g*., neomycin, hydromycin or methotrexate.

The procedures used to ligate the DNA sequences coding the peptides or full-length proteins, the promoter, and the terminator, respectively, and to insert them into suitable vectors containing the information necessary for replication, are well known to persons skilled in the art (*see, e.g.,* Sambrook *et al*., *supra*).

To obtain recombinant peptides, the coding DNA sequences may be fused with a second peptide coding sequence and a protease cleavage site coding sequence, providing a DNA construct encoding the fusion protein, wherein the protease cleavage site coding sequence positioned between the HBP fragment and second peptide coding DNA, inserted into a recombinant expression vector, and expressed in recombinant host cells. In one embodiment, the second peptide comprises glutathion-S-reductase, calf thymosin, bacterial thioredoxin or human ubiquitin natural or synthetic variants, or peptides thereof. In another embodiment, a peptide sequence comprising a protease cleavage site may be the Factor Xa, with the amino acid sequence IEGR, enterokinase, with the amino acid sequence DDDDK, thrombin, with the amino acid sequence LVPR/GS, or *Acharombacter lyticus,* with the amino acid sequence XKX, cleavage site.

The host cell into which the expression vector is introduced may be any cell which is capable of expression of the peptides or full-length proteins, and is preferably a eukaryotic cell, such as invertebrate (insect) cells or vertebrate cells, e.g., *Xenopus laevis* oocytes or mammalian cells, in particular insect and mammalian cells. Examples of suitable mammalian cell lines include, without limitation, the HEK293 (ATCC CRL-1573), COS (ATCC CRL-1650), BHK (ATCC CRL-1632, ATCC CCL-10) and/or CHO (ATCC CCL-61) cell lines. Methods of transfecting mammalian cells and expressing DNA sequences introduced in the cells are described in, *e.g.*, Kaufman and Sharp, J. Mol. Biol. 159, 1982, pp. 601-621; Southern and Berg, 1982, J. Mol. Appl. Genet. 1:327-341; Loyter et al., 1982, Proc. Natl. Acad. Sci. USA 79: 422-426; Wigler et al., 1978, Cell 14:725; Corsaro and Pearson, 1981, in Somatic Cell Genetics 7, p. 603; Graham and van der Eb, 1973, Virol. 52:456; and Neumann et al., 1982, EMBO J. 1:841-845.

In alternative embodiments, fungal cells (including yeast cells) may be used as host cells. Non-limiting examples of suitable yeast cells include cells of *Saccharomyces* spp. or *Schizosaccharomyces* spp., in particular strains of *Saccharomyces cerevisiae.* Examples of other fungal cells are cells of filamentous fungi, *e.g., Aspergillus* spp. or *Neurospora* spp., in particular strains of *Aspergillus oryzae* or *Aspergillus niger.* The use of *Aspergillus* spp. for the expression of proteins is described in, *e.g.,* EP 238 023.

The medium used to culture the cells may be any conventional medium suitable for growing mammalian cells, such as a serum-containing or serum-free medium containing appropriate supplements, or a suitable medium for growing insect, yeast or fungal cells. Suitable media are available from commercial suppliers or may be prepared according to published recipes (*e.g.,* in catalogues of the American Type Culture Collection).

The peptides or full-length proteins recombinantly produced by the cells may then be recovered from the culture medium by conventional procedures including separating the host cells from the medium by centrifugation or filtration, precipitating the proteinaceous components of the supernatant or filtrate by means of a salt, *e.g*., ammonium sulfate, and/or purification by any of a variety of chromatographic procedures, *e.g*., HPLC, ion exchange chromatography, affinity chromatography, or the like.

In other aspects, the peptides (*e.g.,* NCAM peptide mimetics) are produced by use of synthetic production.

Methods for synthetic production of peptides are well known in the art. Detailed descriptions as well as practical advice for producing synthetic peptides may be found in, *e.g*., Synthetic Peptides: A User's Guide (Advances in Molecular Biology), Grant G. A. ed., Oxford University Press, 2002, and in Pharmaceutical Formulation: Development of Peptides and Proteins, Frokjaer and Hovgaard eds., Taylor and Francis, 1999.

In some embodiments, peptides may be synthesized by using Fmoc chemistry and with Acm-protected cysteines. After purification by reversed phase HPLC, peptides may be further processed to obtain, for example, cyclic or C- or N-terminal modified isoforms. The methods for cyclization and terminal modification are well-known in the art and described in detail in the above-cited manuals.

In other embodiments, peptides may be produced synthetically, in particular, by the Sequence Assisted Peptide Synthesis (SAPS) method.

In certain embodiments, peptides may be synthesized either batchwise in a polyethylene vessel equipped with a polypropylene filter for filtration or in the continuous-flow version of the polyamide solid-phase method (Dryland, A. and Sheppard, R. C., (1986) J. Chem. Soc. Perkin Trans. I, 125-137) on a fully automated peptide synthesizer using 9-fluorenylmethyloxycarbonyl (Fmoc) or tert-butyloxycarbonyl, (Boc) as N-a-amino protecting group and suitable common protection groups for side-chain functionalities.

### VI. Purification of Peptides for use in the Invention

The peptides for use in the invention may be purified to substantial purity by standard techniques, including selective precipitation with such substances as ammonium sulfate; column chromatography, immunopurification methods, and others (*see, e.g.,* Scopes, Protein Purification: Principles and Practice (1982); U.S. Patent No. 4,673,641; Ausubel *et al*., *supra;* and Sambrook *et al*., *supra*).

A number of procedures can be employed when recombinant peptides are purified. For example, proteins having established molecular adhesion properties can be reversible fused to recombinant peptides. With the appropriate ligand, the peptides can be selectively adsorbed to a purification column and then freed from the column in a relatively pure form. The fused protein is then removed by enzymatic activity. Finally, the peptide can be purified using immunoaffinity columns.

The purification of proteins from recombinant bacteria as well as standard protein separation techniques for purifying proteins (*e.g.,* solubility fractionation, size differential filtration, column chromatography, *etc.*) are well known in the art and are described in, *e.g.,* U.S. Patent Publication No. 2009/0019557.

### VII. Administration and Pharmaceutical Compositions

In certain aspects, the peptides for use in the invention (NCAM peptide mimetics) are administered directly to a mammalian subject (*e.g.,* human). Administration is by any of the routes normally used for introducing a compound into contact with a tissue to be treated and is well known to those of skill in the art. Although more than one route can be used to administer a particular composition, a particular route can often provide a more immediate and more effective reaction than another route.

In some apsects, the peptides of the present disclosure (*e.g.,* NCAM peptide mimetics) can be combined with other drugs useful for treating neurological conditions or symptoms thereof. In some aspects, the pharmaceutical compositions may comprise an NCAM peptide mimetic combined with at least one additional compound useful for neurological conditions or symptoms thereof, such as those described in, *e.g.,* U.S. Patent Nos. 6,297,262; 6,284,760; 6,284,771; 6,232,326; 6,187,752; 6,117,890; 6,239,162 or 6,166,008.

The pharmaceutical compositions of the invention may comprise a pharmaceutically acceptable carrier. In certain aspects, pharmaceutically acceptable carriers are determined in part by the particular composition being administered, as well as by the particular method used to administer the composition. Accordingly, there is a wide variety of suitable formulations of pharmaceutical compositions of the present invention (*see, e.g.,* REMINGTON'S PHARMACEUTICAL SCIENCES, 18TH ED., Mack Publishing Co., Easton, PA (1990)).

The pharmaceutical compositions of the invention are administered in a manner compatible with the dosage formulation, and in such amount as will be therapeutically effective. The quantity to be administered depends on a variety of factors including, *e.g*., the age, body weight, physical activity, and diet of the subject, the mental disorder to be treated, and the stage or severity of the mental disorder. In certain embodiments, the size of the dose may also be determined by the existence, nature, and extent of any adverse side effects that accompany the administration of a particular compound in a particular subject. In general, the dose equivalent of a compound used in the invention is from about 1 ng/kg to about 10 mg/kg for a typical subject.

In certain embodiments, the dose may take the form of solid, semi-solid, lyophilized powder, or liquid dosage forms, such as, for example, tablets, pills, pellets, capsules, powders, solutions, suspensions, emulsions, suppositories, retention enemas, creams, ointments, lotions, gels, aerosols, foams, or the like, preferably in unit dosage forms suitable for simple administration of precise dosages.

In the practice of this invention, the compositions can be administered, for example, orally, nasally, by inhalation, topically, intravenously, subcutaneously, intraperitoneally, intrathecally, and/or intracerebroventricularly.

As used herein, the term "unit dosage form" refers to physically discrete units suitable as unitary dosages for human subjects and other mammals, each unit containing a predetermined quantity of a peptide calculated to produce the desired onset, tolerability, and/or therapeutic effects, in association with a suitable pharmaceutical excipient (e.g., an ampoule). In addition, more concentrated dosage forms may be prepared, from which the more dilute unit dosage forms may then be produced. The more concentrated dosage forms thus will contain substantially more than, *e.g.,* at least 1, 2, 3, 4, 5, 6, 7, 8, 9, 10, or more times the amount of the peptide.

Methods for preparing such dosage forms are known to those skilled in the art (*see, e.g.,* REMINGTON'S PHARMACEUTICAL SCIENCES, 18TH ED., Mack Publishing Co., Easton, PA (1990)). The dosage forms typically include a conventional pharmaceutical carrier or excipient and may additionally include other medicinal agents, carriers, adjuvants, diluents, tissue permeation enhancers, solubilizers, and the like. Appropriate excipients can be tailored to the particular dosage form and route of administration by methods well known in the art (*see, e.g., REMINGTON'S PHARMACEUTICAL SCIENCES, supra*).

Examples of suitable excipients include, but are not limited to, lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, water, saline, syrup, methylcellulose, ethylcellulose, hydroxypropylmethylcellulose, and polyacrylic acids such as Carbopols, *e.g.,* Carbopol 941, Carbopol 980, Carbopol 981, *etc.* The dosage forms can additionally include lubricating agents such as talc, magnesium stearate, and mineral oil; wetting agents; emulsifying agents; suspending agents; preserving agents such as methyl-, ethyl-, and propyl-hydroxy-benzoates (*i.e.,* the parabens); pH adjusting agents such as inorganic and organic acids and bases; sweetening agents; and flavoring agents. The dosage forms may also comprise biodegradable polymer beads, dextran, and cyclodextrin inclusion complexes.

For oral administration, the therapeutically effective dose can be in the form of tablets, capsules, emulsions, suspensions, solutions, syrups, sprays, lozenges, powders, and sustained-release formulations. Suitable excipients for oral administration include pharmaceutical grades of mannitol, lactose, starch, magnesium stearate, sodium saccharine, talcum, cellulose, glucose, gelatin, sucrose, magnesium carbonate, and the like.

In some embodiments, the therapeutically effective dose takes the form of a pill, tablet, or capsule, and thus, the dosage form can contain, along with a peptide described herein, any of the following: a diluent such as lactose, sucrose, dicalcium phosphate, and the like; a disintegrant such as starch or derivatives thereof; a lubricant such as magnesium stearate and the like; and a binder such a starch, gum acacia, polyvinylpyrrolidone, gelatin, cellulose and derivatives thereof. A peptide can also be formulated into a suppository disposed, for example, in a polyethylene glycol (PEG) carrier.

Liquid dosage forms can be prepared by dissolving or dispersing a peptide and optionally one or more pharmaceutically acceptable adjuvants in a carrier such as, for example, aqueous saline (*e.g.,* 0.9% w/v sodium chloride), aqueous dextrose, glycerol, ethanol, and the like, to form a solution or suspension, *e.g*., for oral, topical, or intravenous administration. A peptide can also be formulated into a retention enema.

For topical administration, the therapeutically effective dose can be in the form of emulsions, lotions, gels, foams, creams, jellies, solutions, suspensions, ointments, and transdermal patches. For administration by inhalation, a peptide can be delivered as a dry powder or in liquid form via a nebulizer. Aerosol formulations can be placed into pressurized acceptable propellants such as dichlorodifluoromethane. For parenteral administration, the therapeutically effective dose can be in the form of sterile injectable solutions and sterile packaged powders. Preferably, injectable solutions are formulated at a pH of from about 4.5 to about 7.5.

The therapeutically effective dose can also be provided in a lyophilized form. Such dosage forms may include a buffer, *e.g*., bicarbonate, for reconstitution prior to administration, or the buffer may be included in the lyophilized dosage form for reconstitution with, *e.g*., water. The lyophilized dosage form may further comprise a suitable vasoconstrictor, *e.g*., epinephrine. The lyophilized dosage form can be provided in a syringe, optionally packaged in combination with the buffer for reconstitution, such that the reconstituted dosage form can be immediately administered to a subject.

### VIII. Example

It is understood that the example and embodiments described herein are for illustrative purposes only.

### Example 1. Pharmacological Studies with NCAM Peptide Mimetics.

This example demonstrates the effects of the administration of NCAM peptide mimetics such as FGLₘ, FGL_{L}, FGLₛ, and plannexin on anxiety and depression in an animal model system.

### FGLₘ

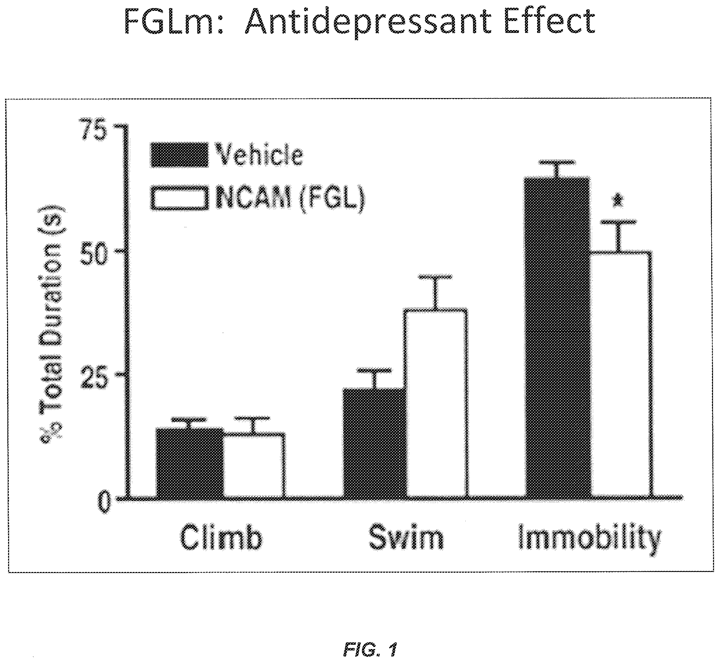

The NCAM peptide mimetic FGLₘ (5 µg) or vehicle was administered intracerebroventricularly (i.c.v.) to adult rats. Figure 1 shows that administration of FGLₘ decreased depression, *e.g*., produced an antidepressant-like effect, as measured by the forced swim test (FST). In particular, animals receiving FGLₘ exhibited significantly more swimming and less immobility compared to vehicle controls.

### FGL_{L}

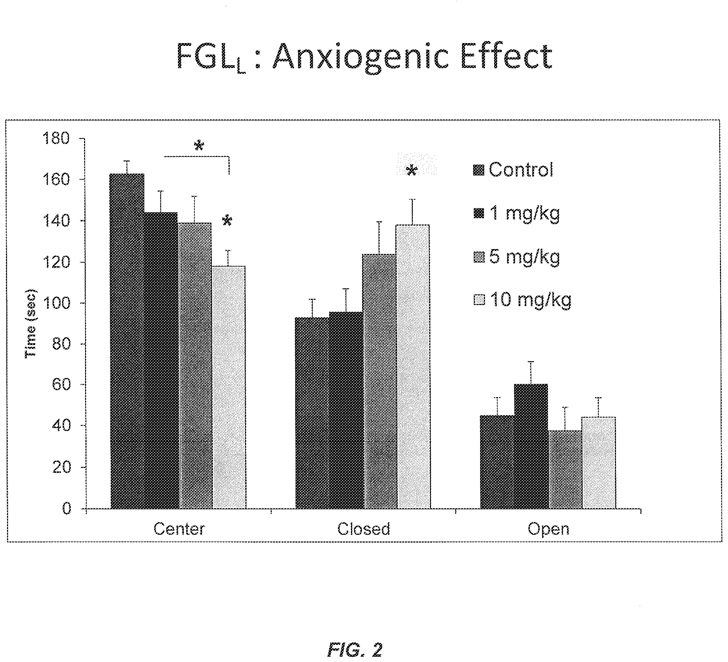

The NCAM peptide mimetic FGL_{L} (1 mg/kg, 5 mg/kg, or 10 mg/kg) or control was administered subcutaneously (s.c.) to adult rats. For the elevated plus maze (EPM), which measures anxiety-like behavior, animals were injected 30 minutes before the test. For the FST, which measures depression-like behavior, animals were injected 1 hour and 5 hours after Day 1 and 30 minutes before Day 2. Figure 2 shows a dose-response curve for FGL_{L} in which higher doses of the peptide mimetic significantly increased the time the animals spent in the "Closed" parts of the EPM. As such, administration of FGL_{L} increased anxiety, *e.g*., produced an anxiogenic effect, as measured by the EPM in an animal model system.

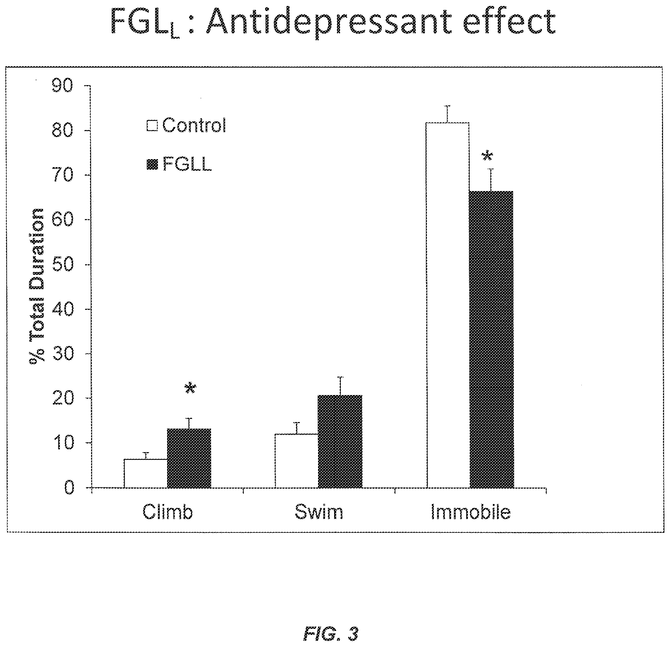

FGL_{L} (10 mg/kg, s.c.) or control was also administered to adult rats via injection every 24 hours for 16 days. Animals were tested for locomotion on Day 13, tested for anxiety-like behavior with the EPM on Day 14, tested for depression-like behavior with the FST on Days 15 and 16, and sacrificed on Day 17. Figure 3 illustrates that FGL_{L} administration decreased depression, *e.g*., produced an antidepressant-like effect, as measured by the FST. In particular, animals receiving FGL_{L} exhibited significantly more climbing and less immobility when compared to controls.

### FGLs

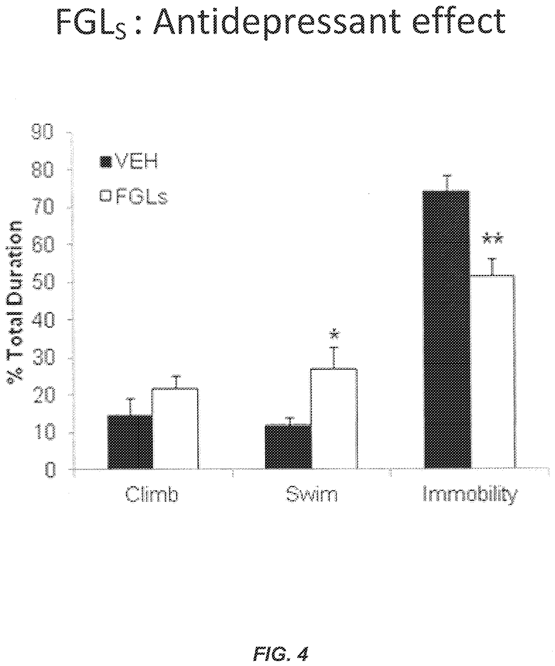

The NCAM peptide mimetic FGLs or vehicle ("VEH") was administered subcutaneously (s.c.) to adult rats and the effect on depression-like behavior was measured by the forced swim test (FST). Animals received 5 injections of 10 mg/kg FGLs or vehicle separated by 1 hour, starting 1 hour after the Day 1 swim. Animals were then given one injection of 10 mg/kg FGLs or vehicle 30 minutes before the Day 2 test. Figure 4 shows that administration of FGLs decreased depression, *e.g*., produced an antidepressant-like effect, as measured by the FST. In particular, animals receiving FGLs exhibited significantly more swimming (p < 0.05) and less immobility (p < 0.005) compared to vehicle controls. These results illustrate that FGLs administration had a statistically significant effect on depression-like behavior.

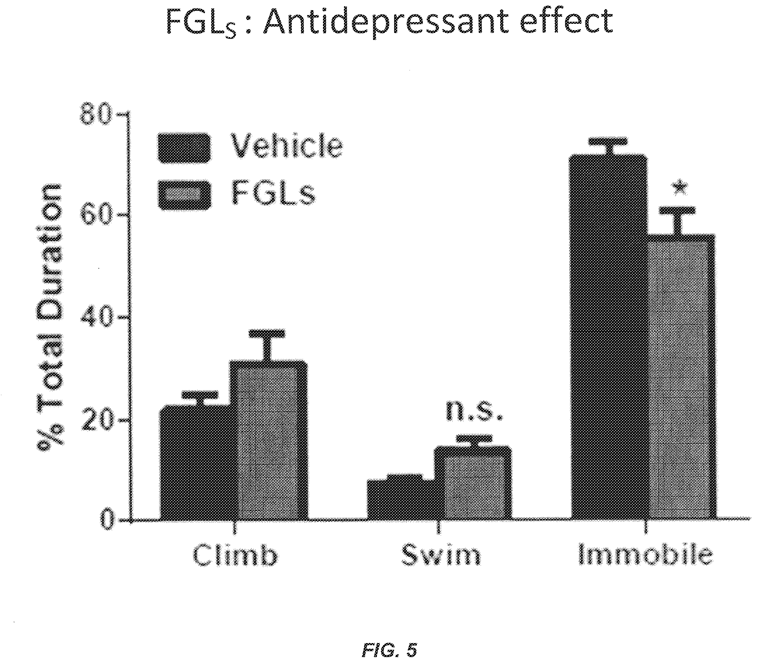

FGLs or vehicle was also administered subcutaneously by an osmotic minipump to adult rats and the effect on depression was measured by the forced swim test (FST). Animals received a total of 12 mg/day of FGLs. The animals were tested for depression-like behavior in the FST on Day 16. As shown in Figure 5, administration of FGLs decreased depression, *e.g*., produced an antidepressant-like effect, as measured by the FST. In particular, animals receiving FGLs exhibited significantly less immobility (p<0.05) compared to vehicle controls. These results show that FGLs administration had a statistically significant effect on depression.

### Plannexin

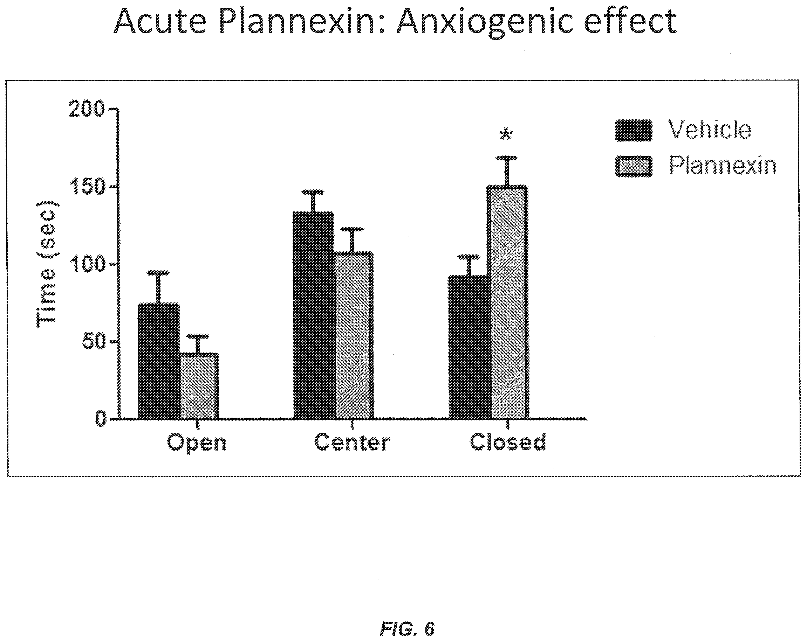

The NCAM peptide mimetic plannexin (10 mg/kg) or vehicle control was acutely administered intraperitoneally (i.p.) to adult rats. Figure 6 shows that acute administration of plannexin significantly increased the time animals spent in the "Closed" parts of the EPM. As such, acute administration of plannexin increased anxiety, *e.g*., produced an anxiogenic effect, as measured by the EPM in an animal model system.

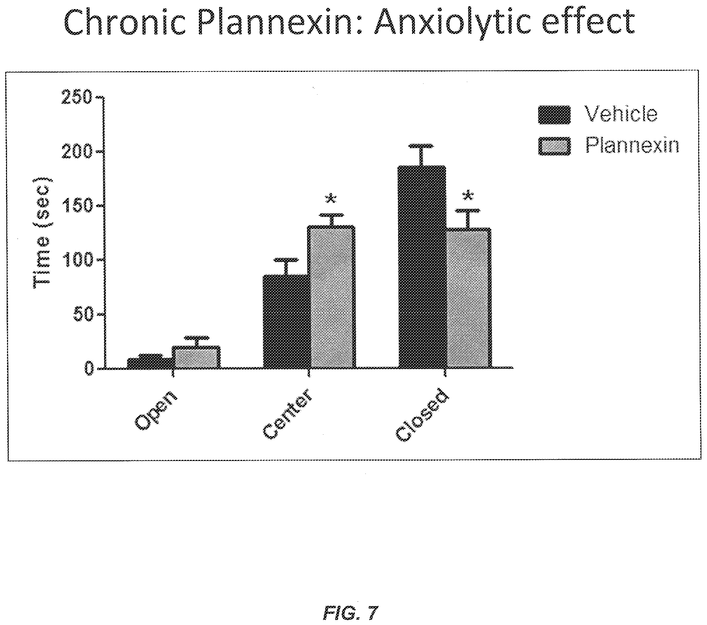

Plannexin (10 mg/kg, i.p.) or control was also chronically administered to adult rats via injection every 48 hours on odd days. Animals were tested for locomotion on Day 13, tested for anxiety-like behavior with the EPM on Day 14, tested for depression-like behavior with the FST on Days 15 and 16, and sacrificed on Day 17. Figure 7 illustrates that chronic administration of plannexin significantly decreased the time animals spent in the "Closed" parts of the EPM and significantly increased the time animals spent in the "Center" of the EPM. As such, chronic administration of plannexin decreased anxiety, *e.g*., produced an anxiolytic effect, as measured by the EPM in an animal model system.

## Claims

1. An NCAM peptide mimetic for use in a method for treating and/or alleviating one or more symptoms of major depression disorder (MDD) in a subject comprising administering a therapeutically effective amount of the NCAM peptide mimetic to the subject, wherein the NCAM peptide mimetic is a compound having the following structure:

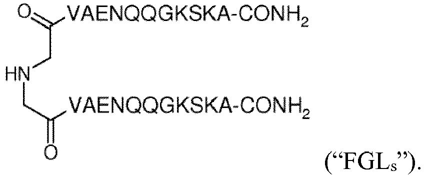

2. The NCAM peptide mimetic for use according to claim 1, wherein the NCAM peptide mimetic is administered with a pharmaceutically acceptable carrier.

3. The NCAM peptide mimetic for use according to claim 1 or 2, wherein the NCAM peptide mimetic is administered via a route selected from orally, nasally, topically, subcutaneously, intravenously, intraperitoneally, intrathecally, intracerebroventricularly, and by inhalation.

4. The NCAM peptide mimetic for use according to any one of claims 1 to 3, wherein the therapeutically effective amount of the NCAM peptide mimetic comprises a dose of between about 0.001 mg/kg to about 1,000 mg/kg per day.

5. The NCAM peptide mimetic for use according to any one of claims 1 to 4, wherein the NCAM peptide mimetic substantially relieves one or more of the symptoms of MDD in the subject for about 1 week or more after the administration.

6. The NCAM peptide mimetic for use according to any one of claims 1 to 5, wherein the NCAM peptide mimetic substantially relieves one or more of the symptoms of MDD in the subject within about 1 day to about 14 days after the administration.

7. The NCAM peptide mimetic for use according to any one of claims 1 to 6, wherein the therapeutically effective amount of the NCAM peptide mimetic is an amount that is sufficient to decrease anxiety (anxiolytic effect) in the subject.

8. The NCAM peptide mimetic for use according to any one of claims 1 to 7, wherein the therapeutically effective amount of the NCAM peptide mimetic is an amount that is sufficient to decrease depression (antidepressant effect) in the subject.

9. The NCAM peptide mimetic for use according to any one of claims 1 to 8, wherein the therapeutically effective amount of the NCAM peptide mimetic is administered acutely.

10. The NCAM peptide mimetic for use according to any one of claims 1 to 8, wherein the therapeutically effective amount of the NCAM peptide mimetic is administered chronically.

## Patentansprüche

1. NCAM-Peptidmimetikum zur Verwendung in einem Verfahren zum Behandeln und/oder Lindern von einem oder mehreren Symptomen von klinischer Depression (major depression disorder, MDD) in einem Subjekt, umfassend ein Verabreichen einer therapeutisch wirksamen Menge des NCAM-Peptidmimetikums an das Subjekt, wobei das NCAM-Peptidmimetikum eine Verbindung mit der folgenden Struktur ist:

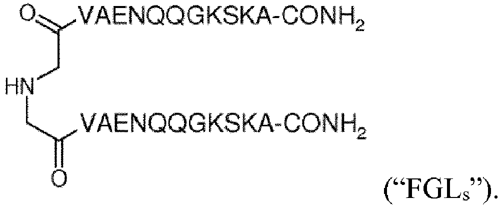

2. NCAM-Peptidmimetikum zur Verwendung nach Anspruch 1, wobei das NCAM-Peptidmimetikum mit einem pharmazeutisch annehmbaren Träger verabreicht wird.

3. NCAM-Peptidmimetikum zur Verwendung nach Anspruch 1 oder 2, wobei das NCAM-Peptidmimetikum über einen Weg verabreicht wird, der aus oral, nasal, topisch, subkutan, intravenös, intraperitoneal, intrathekal, intracerebroventrikulär und durch Inhalation ausgewählt ist.

4. NCAM-Peptidmimetikum zur Verwendung nach einem der Ansprüche 1 bis 3, wobei die therapeutisch wirksame Menge des NCAM-Peptidmimetikums eine Dosis von zwischen ungefähr 0,001 mg/kg bis ungefähr 1.000 mg/kg pro Tag umfasst.

5. NCAM-Peptidmimetikum zur Verwendung nach einem der Ansprüche 1 bis 4, wobei das NCAM-Peptidmimetikum ein oder mehrere Symptome von MDD in dem Subjekt für ungefähr 1 Woche oder mehr nach der Verabreichung wesentlich abschwächt.

6. NCAM-Peptidmimetikum zur Verwendung nach einem der Ansprüche 1 bis 5, wobei das NCAM-Peptidmimetikum ein oder mehrere Symptome von MDD in dem Subjekt innerhalb von ungefähr 1 Tag bis ungefähr 14 Tage nach der Verabreichung wesentlich abschwächt.

7. NCAM-Peptidmimetikum zur Verwendung nach einem der Ansprüche 1 bis 6, wobei die therapeutisch wirksame Menge des NCAM-Peptidmimetikums eine Menge ist, die ausreichend ist, um Angstzustände (anxiolytische Wirkung) in dem Subjekt zu verringern.

8. NCAM-Peptidmimetikum zur Verwendung nach einem der Ansprüche 1 bis 7, wobei die therapeutisch wirksame Menge des NCAM-Peptidmimetikums eine Menge ist, die ausreichend ist, um Depression (antidepressive Wirkung) in dem Subjekt zu verringern.

9. NCAM-Peptidmimetikum zur Verwendung nach einem der Ansprüche 1 bis 8, wobei die therapeutisch wirksame Menge des NCAM-Peptidmimetikums akut verabreicht wird.

10. NCAM-Peptidmimetikum zur Verwendung nach einem der Ansprüche 1 bis 8, wobei die therapeutisch wirksame Menge des NCAM-Peptidmimetikums chronisch verabreicht wird.

## Revendications

1. Mimétique peptidique NCAM pour l'utilisation dans un procédé, pour traiter et/ou réduire un ou plusieurs symptômes de trouble maniacodépressif majeur (MDD) chez un sujet, comprenant l'administration d'une quantité thérapeutiquement efficace du mimétique peptidique NCAM au sujet, dans lequel le mimétique peptidique NCAM est un composé présentant la structure suivante :

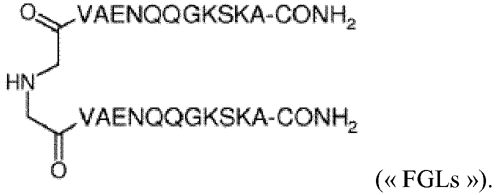

2. Mimétique peptidique NCAM pour l'utilisation selon la revendication 1, dans lequel le mimétique peptidique NCAM est administré avec un vecteur pharmaceutiquement acceptable.

3. Mimétique peptidique NCAM pour l'utilisation selon la revendication 1 ou 2, dans lequel le mimétique peptidique NCAM est administré par l'intermédiaire d'une voie sélectionnée parmi : orale, nasale, topique, sous-cutanée, intraveineuse, intrapéritonéale, intrathécale, intracérébroventriculaire, et par inhalation.

4. Mimétique peptidique NCAM pour l'utilisation selon l'une quelconque des revendications 1 à 3, dans lequel la quantité thérapeutiquement efficace du mimétique peptidique NCAM comprend une dose d'entre environ 0,001 mg/kg et environ 1000 mg/kg par jour.

5. Mimétique peptidique NCAM pour l'utilisation selon l'une quelconque des revendications 1 à 4, dans lequel le mimétique peptidique NCAM soulage sensiblement un ou plusieurs des symptômes de MDD chez le sujet pendant environ 1 semaine ou plus après l'administration.

6. Mimétique peptidique NCAM pour l'utilisation selon l'une quelconque des revendications 1 à 5, dans lequel le mimétique peptidique NCAM soulage sensiblement un ou plusieurs des symptômes de MDD chez le sujet au sein d'une période d'environ 1 jour à environ 14 jours après l'administration.

7. Mimétique peptidique NCAM pour l'utilisation selon l'une quelconque des revendications 1 à 6, dans lequel la quantité thérapeutiquement efficace du mimétique peptidique NCAM est une quantité qui est suffisante pour réduire l'anxiété (effet anxiolytique) chez le sujet.

8. Mimétique peptidique NCAM pour l'utilisation selon l'une quelconque des revendications 1 à 7, dans lequel la quantité thérapeutiquement efficace du mimétique peptidique NCAM est une quantité qui est suffisante pour réduire la dépression (effet antidépresseur) chez le sujet.

9. Mimétique peptidique NCAM pour l'utilisation selon l'une quelconque des revendications 1 à 8, dans lequel la quantité thérapeutiquement efficace du mimétique peptidique NCAM est administrée de façon aiguë.

10. Mimétique peptidique NCAM pour l'utilisation selon l'une quelconque des revendications 1 à 8, dans lequel la quantité thérapeutiquement efficace du mimétique peptidique NCAM est administrée de façon chronique.
